Europäisches Patentamt
European Patent Office
Office européen des brevets

(11) Veröffentlichungsnummer: **0 285 985**
**A1**

# EUROPÄISCHE PATENTANMELDUNG

(21) Anmeldenummer: 88105053.8

(22) Anmeldetag: 29.03.88

(51) Int. Cl.⁴ C07D 417/06 , C07D 417/14 , C07F 9/65 , A01N 43/78 , A01N 57/32

(30) Priorität: 10.04.87 DE 3712307

(43) Veröffentlichungstag der Anmeldung: 12.10.88 Patentblatt 88/41

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Wolf, Hilmar, Dr.
Haus Gravener Strasse 105
D-4018 Langenfeld(DE)
Erfinder: Becker, Benedikt, Dr.
Metzkausener Strasse 14
D-4020 Mettmann(DE)
Erfinder: Homeyer, Bernhard, Dr.
Obere Strasse 28
D-5090 Leverkusen3(DE)
Erfinder: Stendel, Wilhlem, Dr.
In den Birken 55
D-5600 Wuppertal 1(DE)

## (54) 3-Substituierte 1-(2-Chlorthiazol-5-yl-methyl)-2-nitroimino-1,3-diazacycloalkane.

(57) Es werden neue 3-substituierte 1-(2-Chlor-thiazol-5-yl-methyl)-2-nitroimino-1,3-diazacycloalkane der Formel (I)

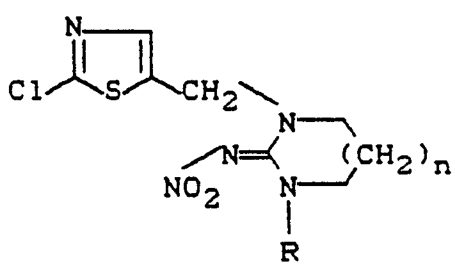

(I)

bereitgestellt, in welcher
n für die Zahlen 0 oder 1 steht und
R für Methyl, Ethyl, Propyl, Isopropyl, Butyl, Isobutyl, sec-Butyl, $C_5$-$C_{20}$-Alkyl, $C_3$-$C_{10}$-Alkenyl, $C_3$-$C_{10}$-Alkinyl oder für eine der Gruppierungen $-CH_2-R^1$, $-COR^2$, $-S(O)_m-R^3$,

EP 0 285 985 A1

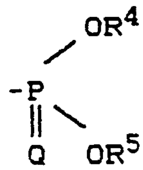

steht, wobei

$R^1$ für jeweils einen gegebenenfalls substituierten Rest der Reihe Phenyl, Pyridyl, Furyl, Thienyl oder Thiadiazolyl steht,

$R^2$ für Methyl steht - mit der Maßgabe, daß dann n für 0 steht -
oder für gegebenenfalls substituiertes $C_2$-$C_{20}$-Alkyl, gegebenenfalls substituiertes $C_3$-$C_6$-Cycloalkyl, gegebenenfalls substituiertes $C_2$-$C_{20}$-Alkenyl, gegebenenfalls substituiertes Phenyl, für Methoxy, $C_3$-$C_{20}$-Alkoxy, Benzyloxy oder Phenoxy steht,

m für die Zahlen 0, 1 oder 2 steht,

$R^3$ für gegebenenfalls substituiertes $C_1$-$C_{20}$-Alkyl oder für gegebenenfalls substituiertes Phenyl steht,

Q für Sauerstoff oder Schwefel steht und

$R^4$ und $R^5$ für $C_1$-$C_4$-Alkyl stehen.

Die Verbindungen der Formel (I) besitzen eine stark ausgeprägte Wirksamkeit gegen tierische Schädlinge, vorzugsweise gegen Arthropoden und Nematoden, insbesondere gegen Insekten und Spinnentiere.

# 3-Substituierte 1-(2-Chlor-thiazol-5-yl-methyl)-2-nitroimino-1,3-diazacycloalkane

Die vorliegende Erfindung betrifft neue 3-substituierte 1-(2-Chlor-thiazol-5-yl-methyl)-2-nitroimino-1.3-diazacycloalkane, ein Verfahren zu ihrer Herstellung und ihre Verwendung in Schädlingsbekämpfungsmitteln, insbesondere als Insektizide.

Es ist bereits bekannt, daß bestimmte organische Nitroverbindungen, wie z. B. 2-Nitromethylen-2H-tetrahydro-1.3-thiazin, insektizide Eigenschaften aufweisen (vgl. US-PS 3 993 648).

Es wurden nun die neuen 3-substituierten 1-(2-Chlor-thiazol-5-yl-methyl)-2-nitroimino-1.3-diazacycloalkane der allgemeinen Formel (I)

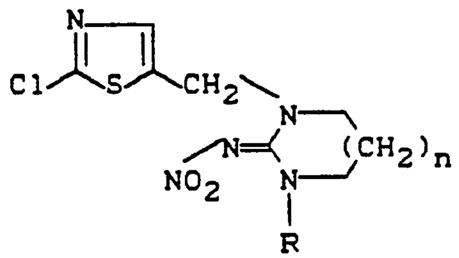

(I)

in welcher

n für die Zahlen 0 oder 1 steht und

R für Methyl, Ethyl, Propyl, Isopropyl, Butyl, Isobutyl, sec-Butyl, $C_5$-$C_{20}$-Alkyl, $C_3$-$C_{10}$-Alkenyl, $C_3$-$C_{10}$-Alkinyl oder für eine der Gruppierungen $-CH_2-R^1$, $-COR^2$, $-S(O)_m-R^3$,

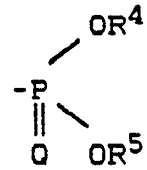

steht, wobei

$R^1$ für jeweils einen gegebenenfalls substituierten Rest der Reihe Phenyl, Pyridyl, Furyl, Thienyl oder Thiadiazolyl steht,

$R^2$ für Methyl steht - mit der Maßgabe, daß dann n für 0 steht -
oder für gegebenenfalls substituiertes $C_2$-$C_{20}$-Alkyl, gegebenenfalls substituiertes $C_3$-$C_6$-Cycloalkyl, gegebenenfalls substituiertes $C_2$-$C_{20}$-Alkenyl, gegebenenfalls substituiertes Phenyl, für Methoxy, $C_3$-$C_{20}$-Alkoxy, Benzyloxy oder Phenoxy steht,

m für die Zahlen 0, 1 oder 2 steht,

$R^3$ für gegebenenfalls substituiertes $C_1$-$C_{20}$-Alkyl oder für gegebenenfalls substituiertes Phenyl steht,

Q für Sauerstoff oder Schwefel steht und

$R^4$ und $R^5$ für $C_1$-$C_4$-Alkyl stehen, gefunden.

Als Substituenten für $R^1$ = gegebenenfalls substituiertes Phenyl kommen vorzugsweise in Frage: Halogen, Cyano, Nitro, $C_1$-$C_4$-Alkyl, Trifluormethyl, $C_1$-$C_4$-Alkoxy, $C_1$-$C_4$-Alkoxycarbonyl.

Als Substituenten für $R^1$ = gegebenenfalls substituiertes Pyridyl kommen vorzugsweise in Frage: Halogen, Cyano, Nitro, $C_1$-$C_4$-Alkyl, Trifluormethyl, $C_1$-$C_4$-Alkoxy, $C_1$-$C_4$-Alkoxycarbonyl.

Als Substituenten für $R^1$ = gegebenenfalls substituiertes Furyl, Thienyl, Thiazolyl oder Thiadiazolyl kommen vorzugsweise in Frage: Halogen, $C_1$-$C_4$-Alkyl, $C_1$-$C_4$-Halogenalkyl.

Als Substituenten für $R^2$ = gegebenenfalls substituiertes $C_2$-$C_{20}$-Alkyl kommen vorzugsweise in Frage: Halogen, Cyano, $C_1$-$C_4$-Alkoxy.

Als Substituenten für $R^2$ = gegebenenfalls substituiertes $C_3$-$C_6$-Cycloalkyl kommen vorzugsweise in Frage: Halogen, $C_1$-$C_4$-Alkyl.

Als Substituenten für $R^2$ = gegebenenfalls substituiertes $C_2$-$C_{20}$-Alkenyl kommen vorzugsweise in Frage: Halogen.

Als Substituenten für $R^2$ = gegebenenfalls substituiertes Phenyl kommen vorzugsweise in Frage:

Halogen, $C_1$-$C_4$-Alkyl, Trifluormethyl, Cyano, Nitro, $C_1$-$C_4$-Alkoxy, $C_1$-$C_4$-Alkoxycarbonyl.

Als Substituenten für $R^3$ = gegebenenfalls substituiertes $C_1$-$C_{20}$-Alkyl kommen vorzugsweise in Frage: Halogen.

Als Substituenten für $R^3$ = gegebenenfalls substituiertes Phenyl kommen vorzugsweise in Frage: Halogen, $C_1$-$C_4$-Alkyl, Trifluormethyl, Cyano, Nitro, $C_1$-$C_4$-Alkoxy, $C_1$-$C_4$-Alkoxycarbonyl.

Weiter wurde gefunden, daß man die neuen 3-substituierten 1-(2-Chlor-thiazol-5-yl-methyl)-2-nitroimino-1,3-diazacycloalkane der allgemeinen Formel (I) erhält, wenn man 1-(2-Chlor-thiazol-5-yl-methyl)-2-nitroimino-1,3-diazacycloalkane der allgemeinen Formel (II)

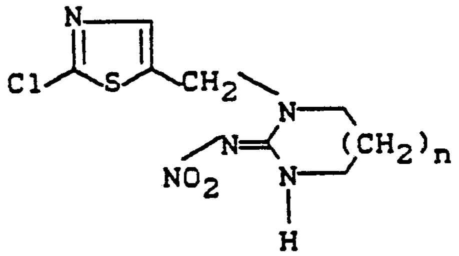

(II)

in welcher

n die oben angegebene Bedeutung hat,

mit Halogenverbindungen der allgemeinen Formel (III)

X - R (III)

in welcher

R die oben angegebene Bedeutung hat und

X für Halogen steht,

gegebenenfalls in Gegenwart eines Säureakzeptors und gegebenenfalls in Gegenwart eines Verdünnungsmittels umsetzt.

Die neuen 3-substituierten 1-(2-Chlor-thiazol-5-yl-methyl)-2-nitroimino-1,3-diazacycloalkane der Formel (I) zeichnen sich durch hohe Wirksamkeit als Insektizide aus. Überraschenderweise zeigen die erfindungsgemäßen Verbindungen der Formel (I) erheblich stärkere insektizide Wirkung als nach Struktur und Wirkprofil vergleichbare organische Nitroverbindungen, wie z. B. 2-Nitromethylen-2H-tetrahydro-1,3-thiazin.

Die Erfindung betrifft vorzugsweise Verbindungen der Formel (I), in welcher

n für Null steht und

R für Methyl, Ethyl, Propyl, Isopropyl, Butyl, Isobutyl, sec-Butyl oder $C_5$-$C_{18}$-Alkyl, für $C_3$-$C_6$-Alkenyl oder $C_3$-$C_4$-Alkinyl steht, oder für die Gruppierung $-CH_2-R^1$ steht, in welcher

$R^1$ für Phenyl [welches gegebenenfalls durch Fluor, Chlor, Methyl, Methoxy oder $C_1$-$C_2$-Alkoxy-carbonyl substituiert ist], Pyridyl [wel ches gegebenenfalls durch Chlor oder Methyl substituiert ist], Furyl, Thienyl oder Thiazolyl [welche gegebenenfalls durch Chlor oder Methyl substituiert sind] steht,

in welcher weiter

R für die Gruppierung $-CO-R^2$ steht, in welcher

$R^2$ für $C_1$-$C_{18}$-Alkyl, $C_2$-$C_{18}$-Alkenyl, Phenyl [welches gegebenenfalls durch Fluor, Chlor, Brom, Methyl, Trifluormethyl, Cyano, Nitro oder Methoxy substituiert ist] oder $C_3$-$C_8$-Alkoxy steht,

in welcher weiter

R für die Gruppierung $-SO_2-R^3$ steht, in welcher

$R^3$ für $C_1$-$C_{18}$-Alkyl [welches gegebenenfalls durch Fluor oder Chlor substituiert ist] oder Phenyl [welches gegebenenfalls durch Fluor, Chlor, Brom, Cyano, Nitro, Methyl, Trifluormethyl, Methoxy, Difluormethoxy, Trifluormethoxy und/oder $C_1$-$C_2$-Alkoxy-carbonyl ist] steht,

in welcher weiter

R für die Gruppierung

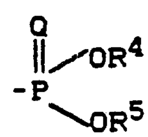

steht, in welcher

Q für Sauerstoff oder Schwefel steht und
$R^4$ und $R^5$ für $C_1$-$C_3$-Alkyl stehen.

Verwendet man zur Durchführung des erfindungsgemäßen Herstellungsverfahrens für die Verbindungen der Formel (I) als Ausgangsstoffe beispielsweise 1-(2-Chlor-thiazol-5-yl-methyl)-2-nitroimino-imidazolidin und Propargylbromid, so kann die Reaktion dieser Verbindungen durch das folgende Formelschema skizziert werden:

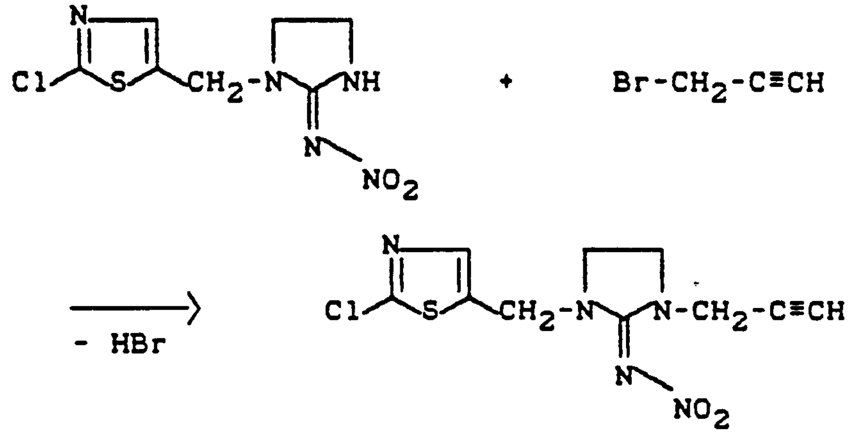

Die beim erfindungsgemäßen Herstellungsverfahren als Ausgangsstoffe zu verwendenden 1-(2-Chlor-thiazol-5-yl-methyl)-2-nitroimino-1.3-diazacycloalkane sind durch die Formel (II) allgemein definiert. In dieser Formel steht n vorzugsweise für Null. Als Ausgangsverbindung der Formel (II) wird somit 1-(2-Chlor-thiazol-5-yl-methyl)-2-nitroimino-imidazolidin bevorzugt.

Die Ausgangsstoffe der Formel (II) sind bereits bekannt (vgl. EP-A 192 060).

Die weiter als Ausgangsstoffe zu verwendenden Halogenverbindungen sind durch die Formel (III) allgemein definiert. In dieser Formel (III) steht R vorzugsweise für diejenigen Reste, die bereits im Zusammenhang mit der Beschreibung der erfindungsgemäßen Stoffe der Formel (I) vorzugsweise für R genannt wurden und X steht vorzugsweise für Chlor oder Brom.

Als Beispiele für die Ausgangsstoffe der Formel (III) seien genannt:
Methyl-, Ethyl-, Propyl-, Isopropyl, Butyl-, Isobutyl-, sec-Butyl-, Pentyl-, Isopentyl-, sec-Pentyl-, Hexyl-, Octyl-, Decyl-, Dodecyl-, Hexadecyl-und Octadecyl-chlorid bzw. -bromid, Allyl-, Crotyl-und Propargyl-chlorid bzw. -bromid, Benzyl-4-Fluor-benzyl-, 4-Chlor-benzyl-, 2-Chlor-benzyl-und 4-Methyl-benzyl-chlorid bzw. -bromid, 2-Chlor-5-chlormethyl-pyridin, 2-Chlor-5-chlormethyl-thiazol, Acetylchlorid, Propionsäure-und Buttersäure-chlorid, Pentansäure-, Hexansäure-, Octansäure-, Decansäure-, Dodecansäure-, Tetradecansäure-, Hexadecansäure-und Octadecansäure-chlorid, Acrylsäure-und Crotonsäure-chlorid, Benzoesäure, 4-Fluor-benzoesäure-, 4-Chlor-benzoesäure-, 4-Nitro-benzoesäure-und 4-Methylbenzoesäure-chlorid, Chlorameisensäure-propylester, -butylester, -pentylester, -hexylester, -heptylester und -octylester, Methan-, Ethan-, Chlormethan-, Trifluormethan-, Tetrafluorbutan-und Perfluoroctan-sulfonsäurechlorid, Benzol-, 2-Fluor-benzol-, 2-Chlor-benzol-, 4-Chlor-benzol-, 2,5-Dichlor-benzol-, 2-Brom-benzol-, 2-Cyano-benzol-, 2-Nitro-benzol-, 4-Nitro-benzol-, 2-Tri fluormethyl-benzol-, 2-Methyl-benzol-, 4-Methyl-benzol-, 2-Methoxy-benzol-, 2-Difluormethoxy-benzol-, 2-Trifluormethoxy-benzol-, 4-Trifluormethoxy-benzol-, 2-Methoxycarbonyl-benzol-, 4-Methoxycarbonyl-benzol-und 2-Ethoxycarbonyl-benzol-sulfonsäurechlorid, Phosphorsäurechlorid-dimethylester und -diethylester sowie Thiophosphorsäure-chlorid-dimethylester und -diethylester.

Die Ausgangsstoffe der Formel (III) sind bekannte chemische Verbindungen.

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl-und Methyl-isobutyl-keton, Ester wie Essigsäuremethylester und-ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren alle üblicherweise für derartige

Umsetzungen verwendbaren Säurebindemittel eingesetzt werden. Vorzugsweise in Frage kommen Alkalimetallhydride wie z. B. Natriumhydrid, Alkalimetallhydroxide, wie z. B. Natrium-und Kaliumhydroxid, Erdalkalihydroxide wie z. B. Calciumhydroxid, Alkalicarbonate und -alkoholate wie Natrium-und Kaliumcarbonat, Natrium-und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4.3.0]-non-5-en (DBN), 1,8-Diazabicyclo-[5.4.0]-undec-7-en (DBU) und 1,4-Diazabicyclo-[2.2.2]-octan (DABCO).

Die Reaktionstemperaturen können bei dem erfindungsgemäßen Verfahren in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren wird im allgemeinen unter Normaldruck durchgeführt.

Zur Durchführung des erfindungsgemäßen Verfahrens werden die Ausganggsstoffe der Formeln (II) und (III) im allgemeinen in angenähert äquimolaren Mengen eingesetzt. Die Ausgangsstoffe werden im allgemeinen bei Raumtemperatur mit dem Säureakzeptor und dem Verdünnungsmittel vermischt und das Reaktionsgemisch wird bei der angegebenen Reaktionstemperatur bis zum Ende der Umsetzung gerührt. Die Aufarbeitung kann nach üblichen Methoden durchgeführt werden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda, z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio

molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe der Formel (I) zeichnen sich durch hervorragende insektizide Wirksamkeit aus. Sie zeigen insbesondere beim Einsatz als Blattinsektizide und Bodeninsektizide hervorragende Wirkung, gegen z. B. gegen Reiszikaden (z. B Nephotettix cincticeps), gegen Blattläuse (z. B. Myzus persicae) und gegen Käferlarven (z. B. Phaedon cochleariae).

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sog. Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens, (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw..

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse

Kieselsäure, Aluminiumoxid und Silikate: als feste Trägerstoffe für Granulate kommen in Frage: z.B. gebrochene und fraktionierte natürliche Ge steine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder -schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene-und Vorratsschädlinge zeichnen sich die Wirkstoffe durch eine hervorragende Residualwirkung auf Holz und Ton sowie durch eine gute Alkalistabilität auf gekälkten Unterlagen aus.

Herstellungsbeispiele

Beispiel 1

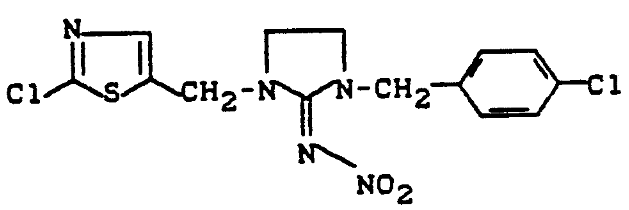

Eine Mischung aus 3,1 g (0,012 Mol) 1-(2-Chlor-thiazol-5-yl-methyl)-2-nitroimino-imidazolidin, 1,9 g (0,012 Mol) 4-Chlor-benzylchlorid, 1,6 g (0,012 Mol) Kaliumcarbonat und 50 ml Acetonitril wird unter Rühren vier Stunden lang zum Rückfluß erhitzt. Nach dem Abkühlen wird filtriert und vom Filtrat wird das Lösungsmittel im Wasserstrahlvakuum abdestilliert. Der verbleibende Rückstand wird mit 50 ml Diethylether verrührt und das kristallin angefallene Produkt durch Absaugen isoliert.

Man erhält 3,65 g (79 % der Theorie) 1-(2-Chlor-thiazol-5-yl-methyl)-2-nitroimino-3-(4-chlor-benzyl)-imidazolidin vom Schmelzpunkt 96 °C.

Analog Beispiel 1 und entsprechend der allgemeinen Beschreibung des erfindungsgemäßen Herstellungsverfahrens können die in der nachstehenden Tabelle aufgeführten Verbindungen der Formel (I) hergestellt werden.

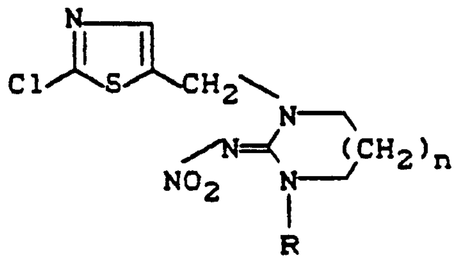

(I)

<u>Tabelle</u>: Weitere Beispiele für Verbindungen der Formel (I)

| Beispiel Nr. | n | -R | Schmelzpunkt (°C) |
|---|---|---|---|
| 2 | 0 | 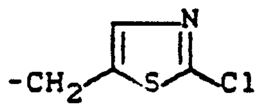 | 90 |
| 3 | 1 | 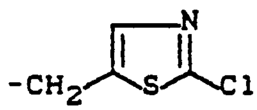 | |
| 4 | 0 | $-CH_2-CH=CH_2$ | (*) |
| 5 | 1 | $-CH_2-CH=CH_2$ | |
| 6 | 1 | 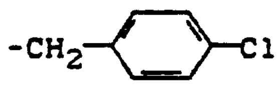 | |
| 7 | 0 | 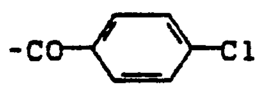 | 165 |
| 8 | 1 | 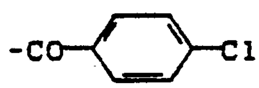 | |
| 9 | 0 | $-SO_2-CH_3$ | 163 |
| 10 | 1 | $-SO_2-CH_3$ | |
| 11 | 0 | $-CO-CH_3$ | 124 |
| 12 | 1 | $-CO-CH_3$ | |
| 13 | 0 | 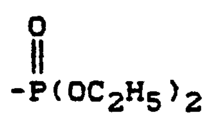 | 103 |

(*)

$\delta_{CH_2}$: 4,62 [ $^1$H-NMR-Signal für 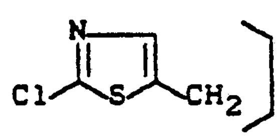 ]

<u>Tabelle</u> - Fortsetzung

| Beispiel Nr. | n | -R | Schmelzpunkt (°C) |
|---|---|---|---|
| 14 | 1 | $-\overset{\overset{O}{\|}}{P}(OC_2H_5)_2$ | |
| 15 | 0 | $-CO-(CH_2)_{10}-CH_3$ | 101 |
| 16 | 1 | $-CO-(CH_2)_{10}-CH_3$ | |
| 17 | 0 | $-(CH_2)_{11}-CH_3$ | 124 |
| 18 | 1 | $-(CH_2)_{11}-CH_3$ | |
| 19 | 0 | $-CO-(CH_2)_{16}-CH_3$ | 102 |
| 20 | 1 | $-CO-(CH_2)_{16}-CH_3$ | |
| 21 | 0 | $-COO-(CH_2)_7-CH_3$ | 73 |
| 22 | 1 | $-COO-(CH_2)_7-CH_3$ | |
| 23 | 0 | $-CH_2-C{\equiv}CH$ | |
| 24 | 1 | $-CH_2-C{\equiv}CH$ | |
| 25 | 0 | $-CH_2$ 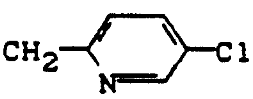 | |
| 26 | 0 | $-CH_2$ 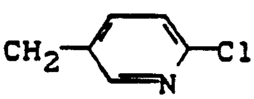 | |
| 27 | 0 | $-CH_2-CH{=}CH-CH_3$ | |
| 28 | 0 | -CO 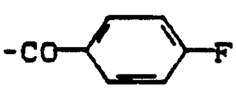 | |
| 29 | 0 | -CO 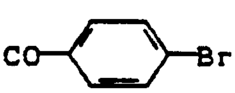 | |

Tabelle - Fortsetzung

| Beispiel Nr. | n | -R | Schmelzpunkt (°C) |
|---|---|---|---|
| 30 | 0 | 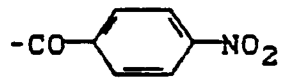 | |
| 31 | 0 | 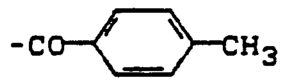 | |
| 32 | 0 | 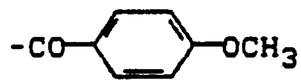 | |
| 33 | 0 | $-SO_2-C_4H_9$ | |
| 34 | 0 | $-SO_2-CH_2Cl$ | |
| 35 | 0 | $-SO_2CF_3$ | |
| 36 | 0 | $-CO-CH(CH_3)_2$ | |
| 37 | 0 | $-CO-C_4H_9$ | |
| 38 | 0 | $-P(=S)(OC_2H_5)_2$ | |
| 39 | 0 | $-CH_3$ | |
| 40 | 0 | $-C_2H_5$ | |
| 41 | 0 | $-CH(CH_3)_2$ | |
| 42 | 0 | $-CH_2-CH(CH_3)_2$ | |
| 43 | 0 | $-C_5H_{11}$ | |
| 44 | 0 | $-C_6H_{13}$ | |
| 45 | 0 | $-COOC_4H_9$ | |

Tabelle - Fortsetzung

| Beispiel Nr. | n | -R | Schmelzpunkt (°C) |
|---|---|---|---|
| 46 | 0 | 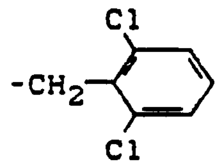 | |
| 47 | 0 | 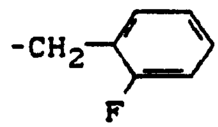 | |
| 48 | 0 | 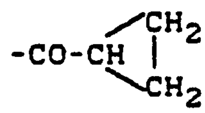 | |
| 49 | 0 | 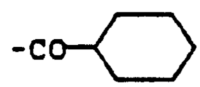 | |
| 50 | 0 | 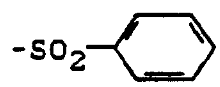 | |
| 51 | 0 | 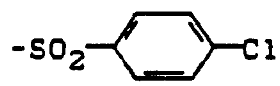 | |
| 52 | 0 | 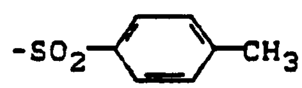 | |

Verwendungsbeispiele

In den nachfolgenden Verwendungsbeispielen wird die nachstehend angegebene Verbindung als Vergleichssubstanz eingesetzt:

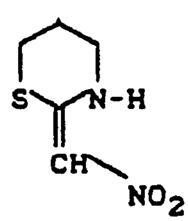

(A)

**2-Nitromethylen-2H-tetrahydro-1,3-thiazin (vgl. US-PS 3 993 648).**

Beispiel A

Nephotettix-Test Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichstteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt. solange die Keimlinge noch feucht sind.

Nach der gewünschten Zeit wird die Abtötung in % bestimmt. Dabei bedeutet 100 %. daß alle Zikaden abgetötet wurden: 0 % bedeutet. daß keine Zikaden abgetötet wurden.

Bei diesem Test zeigen z. B. die gemäß den Herstellungsbeispielen (1). (4). (2). (7). (11). (13). (15) und (21) erhaltenen Verbindungen bei einer Wirkstoffkonzentration von 0.0001 % eine Wirkung von 70 % - 100 % nach 6 Tagen. während die Vergleichssubstanz (A) nur eine Wirkung von 20 % zeigt.

Beispiel B

Myzus-Test Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea). die stark von der Pfirsichblattlaus (Myzus persicae) befallen sind. werden durch Tauchen in die Wirkstoffzubereitung der gewünschten Konzentration behandelt.

Nach der gewünschten Zeit wird die Abtötung in % bestimmt. Dabei bedeutet 100 %. daß alle Blattläuse abgetötet wurden: 0 % bedeutet. daß keine Blattläuse abgetötet wurden.

Bei diesem Test zeigen z. B. die gemäß den Herstellungsbeispielen (1). (2). (4). (11) und (13) erhaltenen Verbindungen bei einer Wirkstoffkonzentration von 0,001 % nach einem Tag eine Wirkung von 60 % - 90 %. während die Vergleichssubstanz (A) nur eine Wirkung von 10 % zeigt.

Beispiel C

Grenzkonzentrations-Test / Wurzelsystemische Wirkung Testinsekt: Phaedon cochleariae-Larven
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test ziegen z. B. die gemäß den Herstellungsbeispielen (1), (2), (4), (7), (11), (13), (15) und (17) erhaltenen Verbindungen bei einer Wirkstoffkonzentration von 20 ppm eine Wirkung von 100 %, während die Vergleichssubstanz (A) keine nachweisbare Wirkung zeigt.

Beispiel D

Grenzkonzentrations-Test Wurzelsystemische Wirkung Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzel systemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z. B. die gemäß den Herstellungsbeispielen (1), (2), (4), (7), (9), (11), (13), (15), (17) und (19) erhaltenen Verbindungen bei einer Wirkstoffkonzentration von 20 ppm eine Wirkung von 100 %, während die Vergleichssubstanz (A) keine nachweisbare Wirkung zeigt.

Beispiel E

Test mit Lucilia cuprina resistent-Larven Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigen z. B. die folgenden Verbindungen der Herstellungsbeispiele überlegene Wirkung gegenüber dem Stand der Technik: (1), (2), (4), (7), (9), (11), (15), (19) und (21).

**Ansprüche**

1. 3-substituierte 1-(2-Chlorthiazol-5-yl-methyl)-2-nitroimino-1,3-diazacycloalkane der allgemeinen Formel (I)

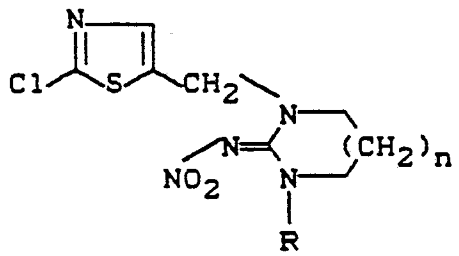

(I)

in welcher

n für die Zahlen 0 oder 1 steht und

R für Methyl. Ethyl. Propyl. Isopropyl. Butyl. Isobutyl, sec-Butyl. $C_5$-$C_{20}$-Alkyl, $C_3$-$C_{20}$-Alkenyl. $C_3$-$C_{20}$-Alkinyl oder für eine der Gruppierungen $-CH_2-R^1$, $-COR^2$, $-S(O)_m-R^3$,

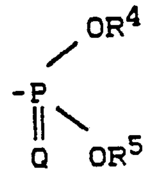

steht, wobei

$R^1$ für jeweils einen gegebenenfalls substituierten Rest der Reihe Phenyl, Pyridyl, Furyl, Thienyl oder Thiadiazolyl steht,

$R^2$ für Methyl steht - mit der Maßgabe, daß dann n für 0 steht

oder für gegebenenfalls substituiertes $C_2$-$C_{20}$-Alkyl, gegebenenfalls substituiertes $C_3$-$C_6$-Cycloalkyl, gegebenenfalls substituiertes $C_2$-$C_{20}$-Alkenyl, gegebenenfalls substituiertes Phenyl, für Methoxy, $C_3$-$C_{20}$-Alkoxy, Benzyloxy oder Phenoxy steht,

m für die Zahlen 0, 1 oder 2 steht,

$R^3$ für gegebenenfalls substituiertes $C_1$-$C_{20}$-Alkyl oder für gegebenenfalls substituiertes Phenyl steht,

Q für Sauerstoff oder Schwefel steht und

$R^4$ und $R^5$ für $C_1$-$C_4$-Alkyl stehen.

2. 3-substituierte 1-(2-Chlorthiazol-5-yl-methyl)-2-nitroimino-1,3-diazacycloalkane der Formel (I) gemäß Anspruch 1),

in welcher

n für die Zahlen 0 oder 1 steht,

R für Methyl, Ethyl, Propyl, Isopropyl, Butyl, Isobutyl, sec-Butyl, $C_5$-$C_{20}$-Alkyl, $C_3$-$C_{20}$-Alkenyl, $C_3$-$C_{20}$-Alkinyl oder für eine der Gruppierungen $-CH_2-R^1$, $-COR^2$, $-S(O)_mR^3$,

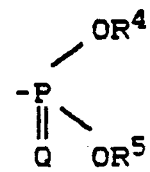

steht, wobei

$R^1$ für gegebenenfalls durch Halogen, Cyano, Nitro, $C_1$-$C_4$-Alkyl, Trifluormethyl, $C_1$-$C_4$-Alkoxy, $C_1$-$C_4$-Alkoxycarbonyl substituiertes Phenyl,

gegebenenfalls durch Halogen, Cyano, Nitro, $C_1$-$C_4$-Alkyl, Trifluormethyl, $C_1$-$C_4$-Alkoxy, $C_1$-$C_4$-Alkoxycarbonyl substituiertes Pyridyl oder für einen gegebenenfalls durch Halogen, $C_1$-$C_4$-Alkyl, $C_1$-$C_4$-Halogenalkyl substituierten Rest der Reihe Furyl, Thienyl, Thiazolyl und Thiadiazolyl steht,

$R^2$ für Methyl steht - mit der Maßgabe, daß dann n für 0 steht - oder für gegebenenfalls durch Halogen, Cyano, $C_1$-$C_4$-Alkoxy substituiertes $C_2$-$C_{20}$-Alkyl, gegebenenfalls durch Halogen, $C_1$-$C_4$-Alkyl substituiertes $C_3$-$C_6$-Cycloalkyl, gegebenenfalls durch Halogen substituiertes $C_2$-$C_{20}$-Alkenyl, gegebenenfalls durch Halogen, $C_1$-$C_4$-Alkyl, Trifluormethyl, Cyano, Nitro, $C_1$-$C_4$-Alkoxy, $C_1$-$C_4$-Alkoxycarbonyl substituiertes Phenyl, Methoxy, $C_3$-$C_{20}$-Alkoxy, Benzyloxy oder Phenoxy steht,

m für die Zahlen 0. 1 oder 2 steht.
$R^3$ für gegebenenfalls durch Halogen substituiertes $C_1$-$C_{20}$-Alkyl oder für gegebenenfalls durch Halogen. $C_1$-$C_4$-Alkyl. Trifluormethyl. Cyano. Nitro. $C_1$-$C_4$-Alkoxy. $C_1$-$C_4$-Alkoxycarbonyl substituiertes Phenyl steht.
Q für Sauerstoff oder Schwefel steht.
$R^4$ und $R^5$ für $C_1$-$C_4$-Alkyl stehen.

3. 3-substituierte 1-(2-Chlorthiazol-5-yl-methyl)-2-nitroimino-1.3-diazacycloalkane der Formel (I) gemäß Anspruch 1).
in welcher
n für Null steht und
R für Methyl. Ethyl. Propyl. Isopropyl. Butyl. Isobutyl. sec.-Butyl. $C_5$-$C_{18}$-Alkyl. $C_3$-$C_6$-Alkenyl. $C_3$-$C_4$-Alkinyl oder für eine der Gruppierungen $-CH_2-R^1$, $-SO_2-R^3$ oder

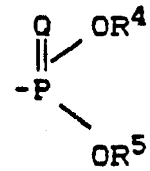

steht. wobei
$R^1$ für gegebenenfalls durch Fluor. Chlor. Methyl. Methoxy oder $C_1$-$C_2$-Alkoxycarbonyl substituiertes Phenyl,
gegebenenfalls durch Chlor oder Methyl substituiertes Pyridyl,
gegebenenfalls durch Chlor oder Methyl substituiertes Furyl,
gegebenenfalls durch Chlor oder Methyl substituiertes Thienyl oder
gegebenenfalls durch Chlor oder Methyl substituiertes Thiazolyl steht,
$R^2$ für gegebenenfalls durch Fluor. Chlor. Brom. Methyl. Trifluormethyl. Cyano. Nitro. Methoxy substituierte Reste der Reihe $C_1$-$C_{18}$-Alkyl, $C_2$-$C_{18}$-Alkenyl, Phenyl steht oder für $C_3$-$C_8$-Alkoxy steht,
$R^3$ für gegebenenfalls durch Fluor oder Chlor substituiertes $C_1$-$C_8$-Alkyl, gegebenenfalls durch Fluor. Chlor. Brom. Cyano. Nitro. Methyl, Trifluormethyl, Methoxy. Difluormethoxy. Trifluormethoxy. $C_1$-$C_2$-Alkoxycarbonyl substituiertes Phenyl steht.
Q für Sauerstoff oder Schwefel steht,
und
$R^4$ und $R^5$ für $C_1$-$C_3$-Alkyl stehen.

4. Verfahren zur Herstellung von 3-substituierten 1-(2-Chlorthiazol-5-yl-methyl)-2-nitroimino-1,3-diazacycloalkanen der allgemeinen Formel (I)

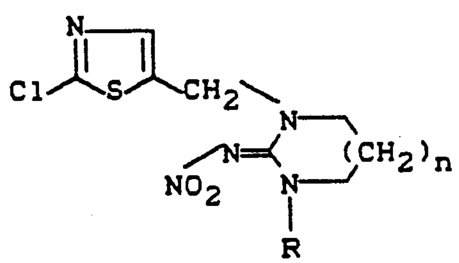
(I)

in welcher
n für die Zahlen 0 oder 1 steht und
R für Methyl. Ethyl. Propyl, Isopropyl, Butyl. Isobutyl, sec-Butyl, $C_5$-$C_{20}$-Alkyl, $C_3$-$C_{10}$-Alkenyl, $C_3$-$C_{10}$-Alkinyl oder für eine der Gruppierungen $-CH_2-R^1$, $-COR^2$, $-S(O)_m-R^3$,

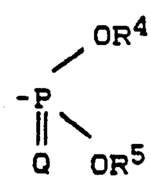

steht, wobei
$R^1$ für jeweils einen gegebenenfalls substituierten Rest der Reihe Phenyl, Pyridyl, Furyl, Thienyl oder Thiadiazolyl steht,
$R^2$ für Methyl steht - mit der Maßgabe, daß dann n für 0 steht
oder für gegebenenfalls substituiertes $C_2$-$C_{20}$-Alkyl, gegebenenfalls substituiertes $C_3$-$C_6$-Cycloalkyl, gegebenenfalls substituiertes $C_2$-$C_{20}$-Alkenyl, gegebenenfalls substituiertes Phenyl, für Methoxy, $C_3$-$C_{20}$-Alkoxy, Benzyloxy oder Phenoxy steht,
m für die Zahlen 0, 1 oder 2 steht,
$R^3$ für gegebenenfalls substituiertes $C_1$-$C_{20}$-Alkyl oder für gegebenenfalls substituiertes Phenyl steht,
Q für Sauerstoff oder Schwefel steht und
$R^4$ und $R^5$ für $C_1$-$C_4$-Alkyl stehen,
dadurch gekennzeichnet, daß man 1-(2-Chlor-thiazol-5-yl-methyl)-nitroimino-1,3-diazacyclo-alkane der allgemeinen Formel (II)

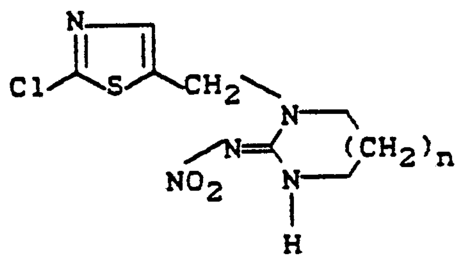

(II)

in welcher
n die oben angegebene Bedeutung hat,
mit Halogenverbindungen der allgemeinen Formel (III)

X - R (III)

in welcher
R die oben angegebene Bedeutung hat und
X für Halogen steht,
gegebenenfalls in Gegenwart eines Säureakzeptors und gegebenenfalls in Gegenwart eines Verdünnungsmittels umsetzt.

5. Schädlingsbekämpfungsmittel, gekennzeichnet durch einen Gehalt an mindestens einem 3-substituierten 1-(2-Chlor-thiazol-5-yl-methyl)-2-nitroimino-1,3-diazacycloalkan der Formel (I).

6. Verfahren zur Bekämpfung von tierischen Schädlingen, dadurch gekennzeichnet, daß man 3-substituierte 1-(2-Chlor-thiazol-5-yl-methyl)-2-nitroimino-1,3-diazacycloalkane der Formel (I) auf tierische Schädlinge und/oder ihren Lebensraum einwirken läßt.

7. Verwendung von 3-substituierten 1-(2-Chlor-thiazol-5-yl-methyl)-2-nitroimino-1,3-diazacycloalkanen der Formel (I) zur Bekämpfung von tierischen Schädlingen.

8. Verfahren zur Herstellung von Mitteln gegen tierische Schädlingen, dadurch gekennzeichnet, daß man 3-substituierte 1-(2-Chlor-thiazol-5-yl-methyl)-2-nitroimino-1,3-diazacycloalkane der Formel (I) mit Streckmitteln und/oder oberflächenaktiven Mitteln vermischt.

Europäisches Patentamt

# EUROPÄISCHER RECHERCHENBERICHT

Nummer der Anmeldung

EP 88 10 5053

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | EP-A-0 192 060 (NIHON TOKUSHU NOYAKU SEIZO K.K.)<br>* Ansprüche *<br>----- | 1-8 | C 07 D 417/06<br>C 07 D 417/14<br>C 07 F 9/65<br>A 01 N 43/78<br>A 01 N 57/32 |
| | | | **RECHERCHIERTE SACHGEBIETE (Int. Cl.4)** |
| | | | C 07 D 417/00<br>C 07 F 9/00 |

Der vorliegende Recherchenbericht wurde für alle Patentansprüche erstellt

| Recherchenort | Abschlußdatum der Recherche | Prüfer |
|---|---|---|
| DEN HAAG | 12-07-1988 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)